# EUROPEAN PATENT SPECIFICATION

(11) **EP 2 809 370 B1**
(45) Date of publication and mention of the grant of the patent: **26.08.2020**
(21) Application number: 13744401.4
(22) Date of filing: 28.01.2013
(51) Int. Cl.: A61L 31/10, A61L 27/34, A61L 27/30, A61L 31/08, A61F 2/82

(54) **NEGATIVELY CHARGED VASCULAR STENT**
NEGATIV GELADENER VASKULÄRER STENT
ENDOPROTHÈSE VASCULAIRE CHARGÉE NÉGATIVEMENT

(30) Priority: 30.01.2012 US 201261592013 P; 24.07.2012 US 201261674861 P
(43) Date of publication of application: 10.12.2014
(73) Proprietor: Ameber Medical LTD, Jerusalem 9695801 (IL)
(72) Inventor: FISHMAN, Michael, 78751 Ashkelon (IL); TSUN, Alexander, 77643 Ashdod (IL)
(74) Representative: Cornuejols, Georges
(86) International application number: PCT/IL2013/050074
(87) International publication number: WO 2013/114358

(56) References cited:
- WO-A1-2011/094459
- WO-A1-2011/109447
- WO-A1-2011/109447
- WO-A2-2011/156603
- US-A- 5 759 205
- US-A1- 2008 140 187
- US-A1- 2009 211 076

## Description

### FIELD OF THE INVENTION

The invention relates to medical devices for mitigating thrombosis risk and more specifically to a stent carrying a stable negative charge.

### BACKGROUND

A stent is an artificial 'tube' inserted into a natural passage/conduit in the body to prevent, or counteract, a disease-induced, localized flow constriction. The stents are inserted into narrowed coronary arteries to help keep them open after balloon angioplasty. The stent then allows the normal flow of blood and oxygen to the heart. Stents placed in narrowed carotid arteries (the vessels in the front of the neck that supply blood to the brain) appear useful in treating patients at elevated risk for stroke.

WO/1996/041589 discloses a device for implantation in a vessel or hollow organ lumen in a human or animal body, such as a self-expanding stent, a cava filter, an embolizing means or a supporting means, comprises a wire frame with a plurality of interconnected cells made of at least two separate wire sections which are intercrossing at cell junctions and form closed cells. At the cell junctions the wires are knot to form a geometrical locking of the cells so that the wire-shaped cell sides in respective cells are locked at the cell junctions when the wire frame is subjected to pressure acting radially inwards.

RU 2446775 discloses a stent includes a tubular element which has longitudinal axis. Tubular element contains first radial element and second radial element. First radial element contains elongated bridge with deflectable teeth. Bridge is characterized by upper and lower parts. Upper part narrows from first thickness to second thickness on first direction on circumference, approaching far edge of upper part. Lower part narrows from first thickness to second thickness in direction on circumference opposite to first direction, approaching far edge of lower part. Second radial element is located on circumference near first radial element and contains gearing means, which have configuration for sliding gearing of elongated bridge of first radial element and deflection of deflectable teeth, when teeth contact with gearing means in such a way that tubular element reaches extension in direction on circumference with reduced kickback. Gearing means narrow to second thickness in direction on circumference, approaching far edge of gearing means. In second version of stent implementation gearing means of second radial element include slot intended for sliding gearing of first radial element bridge and deflection of teeth, when elongated bridge of first radial element passes through slot, in such a way that tubular element obtains extension in direction on circumference with reduced kickback. Second radial element does not overlap itself when stent is in extended state. Gearing means narrow on circumference to second thickness, approaching far edge of gearing means in such a way, that first and second radial elements preserve mainly constant common thickness, when first radial element is geared by gearing means of second radial element and stent is in extended state.

US 6231600 discloses a device such as a stent provided with a hybrid coating including a time released, restenosis inhibiting coating and a non-thrombogenic coating to prevent clotting on the device. One first coat or layer includes a polymer, a crosslinking agent, and pacitaxel, analogues, or derivatives thereof. The first coat preferably includes a polymer having Taxol admixed therein so as to be releasable over time. The first coat preferably includes a polyfunctional aziridine as the crosslinking agent. The second coat preferably includes heparin to inhibit clot formation on the device. The crosslinking agent can covalently bond to both the first coat polymer and the second coat heparin. A stent can be provided with a first coat including an aqueous dispersion or emulsion of a polymer and an excess of crosslinking agent. The first coating can be dried, leaving a water insoluble polymer coating. A second aqueous coating including a solution or dispersion of heparin can be applied over the first coating, the heparin becoming covalently bound to the crosslinking agent on the first coating surface. The resulting stent can inhibit restenosis while preventing blood clot formation on the stent.

RU 2380059 discloses a stent coating containing a polymer material with an active antiproliferative substance. The polymer material presents a copolymer of butyric and valeric acids, while the active antiproliferative substance is rubomycinum. The amount of the copolymer of butyric and valeric acids per one stent is 2-15 mg/stent, while rubomycinum composes a polymer layer in amount 0.002-0.025 mg/stent. US Patent Application 2008/0208315 disclosed a stent for coronary vessels, having a surface of multilayer immobilized structures, includes a stent body and a number of polyelectrolyte complex (PEC) layers stacking and being immobilized on the surface of the stent body, in which the PEC layer is formed of a polymer layer and an anticoagulant layer. The coronary stent is capable of effectively improving the hemocompatibility and longevity over a conventional stent using surface encapsulation of an anticoagulant layer for hemocompatibility improvement. Furthermore, the coronary stent can be use as a drug-eluting coronary stent, thus allowing for the time-releasing of drugs, and further preventing the thickening of vascular smooth muscle cells for causing vascular thrombosis.

It is known in the prior art that blood cells being components of thrombosis such as thrombocytes and leucocytes are negatively charged. US Patent Application
2006/0106451 discloses an electronic anti-coagulation stent structure. The stent structure comprises a pair of coaxial metal stents having a layer of dielectric material between the stents. A battery is operatively connected preferably near or adjacent to the upstream end upon deployment of the stent. The positive battery terminal establishes an electrical connection to the outer metal stent and the negative terminal establishes an electrical connection to the inner metal stent and this exhibits a capacitor-like properties. The inner metal stent, being negatively charged, promotes a platelet repellent, anti-thrombotic effect.

US Patent Application 2011/0196478 discloses devices and methods for lumen treatment. There is provided an endoprosthesis that includes an internal layer designed to provide a negative electric field directed endoluminally; an external layer designed to provide a positive electric field directed exoluminally; and one or more intermediate layers disposed between the internal layer and the external layer, wherein the negative electric field is due to a negative point charge between about -25 mV and about -250 mV, and wherein the positive electric field is due to a positive point charge between about +1 mV and about +30 mV.

WO2011/109447 discloses an internal medical device for implantation into a human or animal where the internal medical device has an electric field source that may be an active source of electromotive force such as a battery or capacitor, or may be an electric field generating material such as an electret, piezoelectric, or the like.

Life span of the battery limits duration of the stent use. When exhausted, the battery should be replaced by means of a surgical procedure. The vascular stents known in the art are characterized by substantive hydrophilicity. Thrombogenic blood particles freely penetrate into the gaps between stent elements or between the stent and vascular walls and aggregate into a new clot. The chances that surgically treated blood vessels will be re-stenosed are very good. Thus, there is a long- felt and unmet need to provide a hydrophobic vascular stent which is able to repel the aforesaid thrombogenic blood particles and prevents the surgically treated blood vessels from recurrent stenosis and sclerosis.

### SUMMARY OF THE INVENTION

It is hence one object of the invention to disclose a stent insertable into a patient's body, said stent comprising a wire member coated with a coating having an electret structure; wherein said electret coating comprises a tantalum pentoxide layer blanketing an entire surface of said wire member.

A further object of the invention is to disclose the tantalum pentoxide coating which is vacuum-plasma sputtering.

A further object of the invention is to disclose the stent in which a distance between each two neighboring wires of said stent is less than 15 wire diameters such that said stent provides substantially uniform electrostatic field preventing blood cells from aggregation on said mesh elements.

A further object of the invention is to disclose the article subject of the invention being expandable such that mesh elements are exposed to electret coating without shading.

A further object of the invention is to disclose a method of manufacturing a stent carrying a negative charge and insertable into a patient's body; said method comprising the steps of:
a. providing a wire member;
b. electret coating said wire member; wherein said step of electret coating said wire member further comprises coating said wire member with a layer of tantalum pentoxide blanketing an entire surface of said wire member.

In particular embodiments, said work piece is mounted on a massive base plate providing effective heat sinking.

In particular embodiments, said article work piece is selected from the group consisting of a wire stent and a mesh stent cut of a metal tubing and wherein said step of coating said wire members further comprises creating an integral surface blanketing an entire surface of said wire member.

### BRIEF DESCRIPTION OF THE DRAWINGS

In order to understand the invention and to see how it may be implemented in practice, a plurality of embodiments is adapted to now be described, by way of non-limiting example only, with reference to the accompanying drawings, in which
Figs 1a and 1b are general views of a wire wound stents;
Fig. 1c is a general view of a stent braided of a wire;
Fig. 1d is a general view of a stent cut of a tube by a laser;
Fig. 2a is an exemplar view of a mesh structure which is cut by a laser;
Fig. 2b is an exemplar view of a mesh structure formed of wavelike wires;
Fig. 2c is an exemplar view of a mesh structure formed of interlaced wires;
Fig. 3a is an illustration specifying aggregation of blood particles into a clot; and
Fig. 3b is an illustration specifying prevention of blood particles from aggregation into a clot.

### DETAILED DESCRIPTION OF THE INVENTION

The following description is provided, so as to enable any person skilled in the art to make use of said invention and sets forth the best modes contemplated by the inventor of carrying out this invention.

The core objective of the present invention is to reduce the risk of recurrent thrombosis. The vascular stent of the present invention is characterized high biological compatibility including hemocompatibility and temporally stable anti-coagulating and anti-clotting properties implantable into coronary or other arteries to restore patency thereof. A vascular stent constituting a cylindrical celled frame made of at least one wire member is coated with a coating having an electret structure. The aforesaid coating carries negative surface charge. It should be emphasized that the electret coating has an integral surface blanketing an entire surface of the stent. In other words, there is a negative potential on entire surface of the stent. Hence, external stent surfaces directed to the vessel wall and internal stent surfaces directed to an internal space thereof carry a negative electric charge. According to one embodiment of the present invention, the electret structure is formed by means of coating the stent with tantalum pentoxide (Ta2Os).

Negative charge carried by the vascular stent coated with electret films prevents blood plates such as thrombocytes or leucocytes from aggregation at a stent mounted within a blood vessel. It is known that the blood cells to be aggregated at the stent are negatively charged. Electronegativity of the stent surface results in prevention of blood cell aggregation both at the stent per se and the vascular wall-adjacent area due to repelling Coulomb forces. Thus, the negative surface charge generates electric static field shielding areas potentially prone to blood cell aggregation. The vascular stent carrying the electret coating decreases risk of restenosis. Life quality and longevity of the patients stented with temporally stable anti-coagulating and anti-clotting properties are improved in comparison with the unstented patient. Doses of anti-coagulating and anti-clotting medicine are cut as well. In accordance with common practice in the relevant field of technology, the stent is expanded due to plastic deformation when mounted within the blood vessel, for example, by means of an inflatable balloon. At least one wire member is configured into a cylindrical celled structure. The aforesaid wire member is coated with tantalum pentoxide. In accordance with one embodiment of the present invention, the tantalum pentoxide coating is made by means of vacuum-plasma sputtering.

The stents provided with tantalum coating are characterized by higher reagent resistance in comparison with the gold coated stents. Tantalum pentoxide is dielectric and characterized by high mechanical properties. Tantalum pentoxide is chemically inert and biologically compatible. Thus, the stent provided with tantalum pentoxide coating is safely implantable into blood vessel and may reside in situ for a long time without expected side effects such as toxicity from stent degradation products and repeat thrombosis at the stent.

The stent with tantalum oxide coating having electret structure is characterized by:
1. Tantalum oxide coating which is coated by means of vacuum-plasma sputtering has improved adhesion to a metallic surface due to the high-energy coating process providing covalent and ionic bondings between a material to be coated and plasma-spattered coating.
2. Negative charge of the electret film can be temporally stable for a predetermined period of time. It is known in the art that the time characteristic can be varied by means of changes in the technological process.
3. It is very important that the stent with tantalum oxide coating can sterilized at high temperature.
4. The tantalum pentoxide coating is characterized by essential radiopacity. No additional efforts should be made for X-ray reliable detectability of the stent coated with tantalum pentoxide.

In accordance with the present invention, at least one wire member is coated with tantalum pentoxide, and then the obtained coated wire member(s) is configured into a stent expandable due to plastic deformation. It should be emphasized that local discontinuances in integrality of tantalum pentoxide coating result in local occurrence of positive electric potentials. As mentioned above, the blood cells which are aggregated in thrombosis are negatively charged. Thus, discontinuances in coating integrality will be centers of clottage/thrombosis. The coating process of the entire stent is very problematic. Specifically, adhesion of the coating in intertangling areas is much lower than the adhesion at linear areas. In accordance with the proposed innovation, wire member is configured into the celled structure after coating with tantalum pentoxide. In the present technical solution, adhesion value does not depend on a position on the stent and has a constant value in any location on the stent. Thus, there are no discontinuances in the coating integrality, and positive potential does not occur in any location. The entire stent is negatively charged and prevents any clottage/thrombosis thereon.

Technological improvement required for practical realization of the present invention is minor because a metal wire used for producing vascular stents should be replaced with a wire coated with an electret coating. Thus, there is no need to change mass production machines producing the vascular stents.

The negatively charged stent can be made of a number of wire members which are coated with tantalum pentoxide before. In this case, impermeability of the coating is not broken, and the positive charge does not occur at the stent surface, because coating integrality of each wire member is kept during a process of stent fabrication.

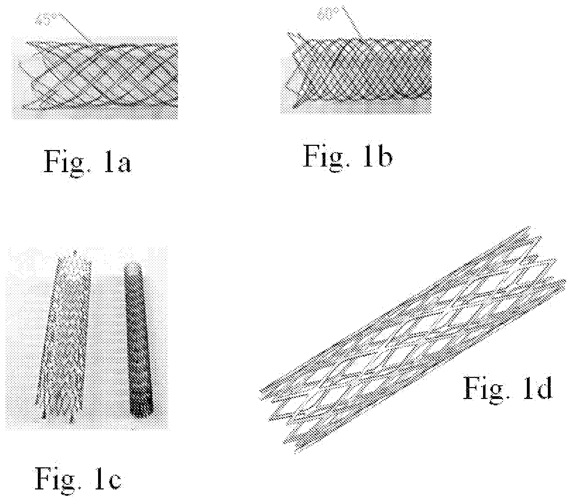

Reference is now made to Figs la to 1d illustrating general views of commercially available stents. Specifically, Fig. 1a and Fig. 1b show wire wound stents. Figs 1c and Fig. 1d illustrate a braided stent and a mesh-like stent laser cut of a tube, respectively.

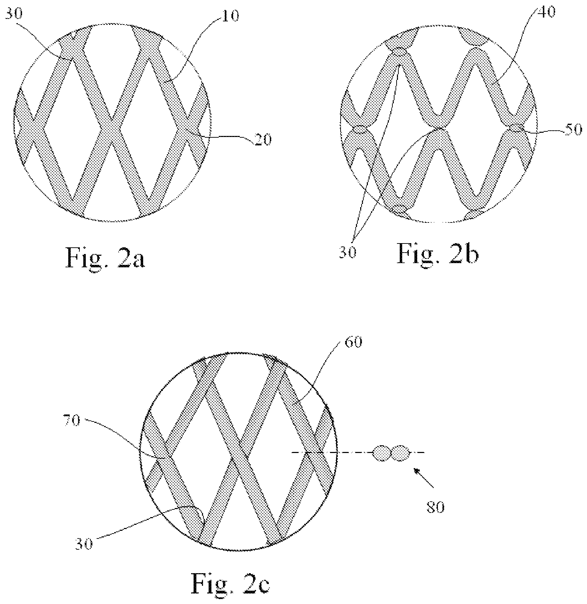

Reference is now made to Fig. 2a, presenting an exemplar mesh-like structure made by means of laser cutting. Numerals 10 and 20 refer to a wire element and a node, respectively. Experiments proved that locations 30 and similar having the mesh elements 10 meeting together at the node 20 at an acute angle serve as points of clot formation.
Reference is now made to Fig. 2b, showing an exemplar mesh-like structure made of wavelike wire elements 40 which are secured to each other by means of spot welding 50. As seen in the Fig. 2b, the locations 30 can be characterized as dangerous in terms of clot formation.

Reference is now made to Fig. 2c, presenting an exemplar mesh constituting an interlaced wire structure. Wires 60 are interlaced in locations 70 which are points of clot formation. A numeral 80 refers to a cross-sectional view of the interlaced wires 60.

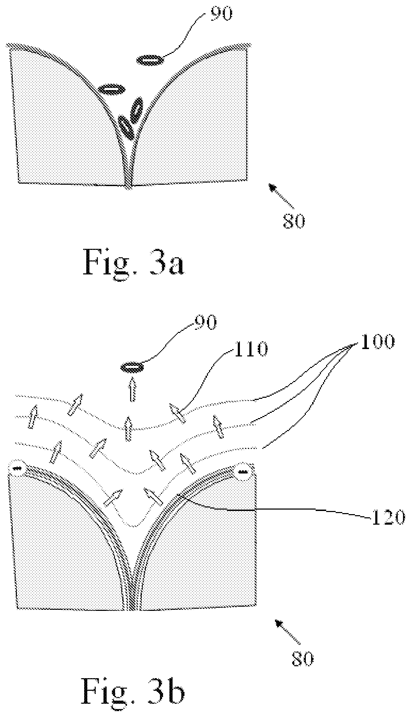

Reference is now made to Fig. 3a which is an illustration specifying aggregation of blood particles into a clot. Specifically, thrombocytes 90 aggregate in proximity of interlaced wires and form a clot.

Reference is now made to Fig. 3b, showing claimed prevention of the thrombocytes from aggregation in proximity of dangerous locations. As known in the art, the thrombocytes carry negative electric charge. An electret coating also carries a permanent negative charge and repels the thrombocytes from the dangerous locations and hence prevents the thrombocytes from aggregation in proximity of the stent. Equipotential lines 100 characterize electric field intensity in proximity of the interlaced wires. A direction of a repelling force acting onto the thrombocytes 90 is indicated by a numeral 1 10.

It should be emphasized that the method of manufacturing the claimed article comprises a step of expanding the article to be coated to prevent shaded areas to provide an integral surface blanketing an entire surface of said wire member. The expanded stent is mounted on a massive base plate providing effective heat sinking to prevent the stent to be coated from overheating during the coating process.

In accordance with a preferred embodiment of the present invention, in any cross section of the vascular stent, a distance between two neighboring wires is less 15 wire diameters. The described stent arrangement provides substantially uniform electrostatic field preventing blood cells from aggregation on the stent wires.

In accordance with another the present invention, the article is a vascular stent.

A vascular stent carrying a surface negative charge is disclosed. The aforesaid stent comprises at least one metal wire member configured into a cylindrical celled frame.

The wire member is coated with a coating having an electret structure.

The coating has an integral surface blanketing an entire surface of said wire member.

The vascular stent is constructed of tubing cut into a mesh shape.

The vascular stent is constructed of at least one wire into a mesh shape.

The coating is tantalum pentoxide.

The tantalum pentoxide coating is vacuum-plasma sputtering.

In accordance with a further the present invention, a method of of manufacturing a stent carrying a negative charge and insertable into a patient's body; said method comprising the steps of:
a. providing a wire member;
b. electret coating said wire member; wherein said step of electret coating said wire member further comprises coating said wire member with a layer of tantalum pentoxide blanketing an entire surface of said wire member.

The step of coating said wire members further comprises sputtering tantalum pentoxide.

The step of sputtering tantalum pentoxide is performed by means of vacuum-plasma sputtering.

In accordance with a preferred embodiment of the present invention, in any cross section of the vascular stent, a distance between two neighboring wires is less 15 wire diameters. The described stent arrangement provides substantially uniform electrostatic field preventing blood cells from aggregation on the stent wires.

The article is a vascular stent.

A vascular stent carrying a surface negative charge is disclosed. The aforesaid stent comprises at least one metal wire member configured into a cylindrical celled frame.

The wire member coated with a coating having an electret structure.

The coating has an integral surface blanketing an entire surface of said wire member.

The vascular stent is constructed of tubing cut into a mesh shape.

The vascular stent is constructed of at least one wire into a mesh shape.

The coating is tantalum pentoxide.

The tantalum pentoxide coating is vacuum-plasma sputtering.

## Claims

1. A stent insertable into a patient's body, said stent comprising a wire member coated with a coating having an electret structure; wherein said electret coating comprises a tantalum pentoxide layer blanketing an entire surface of said wire member.

2. The stent according to claim 1, which is a vascular stent.

3. The stent according to claim 1, wherein said tantalum pentoxide coating is made by vacuum-plasma sputtering method.

4. The stent according to claim 1, wherein said coating comprises Polytetrafluoroethylene.

5. The stent according to claim 2, wherein said vascular stent is constructed of tubing cut into a mesh shape.

6. The stent according to claim 2, wherein said vascular stent is constructed of at least one wire configured into a mesh shape.

7. The stent according to claim 6, wherein a distance between each two neighboring wires of said stent is less than 15 wire diameters such that said stent provides substantially uniform electrostatic field preventing blood cells from aggregation on said mesh elements.

8. The stent according to one of claims 6 or 7 being expandable such that mesh elements are exposed to electret coating without shading.

9. A method of manufacturing a stent carrying a negative charge and insertable into a patient's body; said method comprising the steps of:
a. providing a wire member;
b. electret coating said wire member;
wherein said step of electret coating said wire member further comprises coating said wire member with a layer of tantalum pentoxide blanketing an entire surface of said wire member.

10. The method according to claim 9, wherein said wire member is mounted on a massive base plate providing effective heat sinking.

11. The method according to claim 10, wherein said stent is a vascular stent.

12. The method according to claim 10, wherein said wire member is selected from the group consisting of a wire stent and a mesh stent cut of a metal tubing and wherein said step of coating said wire member further comprises creating an integral surface blancketing the entire surface of said wire member.

13. The method according to claim 10, wherein said step of sputtering tantalum pentoxide is performed by means of vacuum-plasma sputtering.

14. The method according to claim 10 , wherein said step of coating said wire members further comprises coating with Polytetrafluoroethylene.

## Patentansprüche

1. In einen Patientenkörper einführbarer Stent, wobei der genannte Stent ein Drahtelement umfasst, das mit einer Beschichtung mit einer Elektret-Struktur beschichtet ist; wobei die genannte Elektret-Beschichtung eine Tantalpentoxid-Schicht umfasst, die eine gesamte Oberfläche des genannten Drahtelements bedeckt.

2. Stent gemäß Anspruch 1, der ein vaskulärer Stent ist.

3. Stent gemäß Anspruch 1, wobei die genannte Tantalpentoxid-Beschichtung durch ein Vakuumplasma-Zerstäubungsverfahren hergestellt ist.

4. Stent gemäß Anspruch 1, wobei die genannte Beschichtung Polytetrafluorethylen umfasst.

5. Stent gemäß Anspruch 2, wobei der genannte vaskuläre Stent aus Schlauchmaterial konstruiert ist, das aus einer Netzform ausgeschnitten ist.

6. Stent gemäß Anspruch 2, wobei der genannte vaskuläre Stent wenigstens aus einem Draht konstruiert ist, der in einer Netzform ausgestaltet ist.

7. Stent gemäß Anspruch 6, wobei eine Entfernung zwischen jedem zweiten benachbarten Draht des genannten Stents weniger als 15 Drahtdurchmesser derart beträgt, dass der genannte Stent im Wesentlichen ein einheitliches elektrostatisches Feld bereitstellt, das Blutzellen an der Verdichtung auf den genannten Netzelementen gehindert sind.

8. Stent gemäß irgendeinem der Ansprüche 6 oder 7, der derart ausdehnbar ist, dass die Netzelemente der Elektret-Beschichtung ohne Nuancierung freigelegt sind.

9. Herstellungsverfahren eines Stents, der eine negative Ladung trägt und in den Körper eines Patienten einführbar ist; wobei das genannte Verfahren die Schritte umfasst:
a. Bereitstellen eines Drahtelements;
b. Elektret-Beschichtung des genannten Drahtelements;
wobei der genannte Schritt der Elektret-Beschichtung des genannten Drahtelements weiterhin das Beschichten des genannten Drahtelements mit einer Schicht aus Tantalpentoxid umfasst, die eine ganze Oberfläche des genannten Drahtelements bedeckt.

10. Verfahren gemäß Anspruch 9, wobei das genannte Drahtelement auf einer massiven Basisplatte montiert ist, die eine wirkungsvolle Wärmereduzierung bereitstellt.

11. Verfahren gemäß Anspruch 10, wobei der genannte Stent ein vaskulärer Stent ist.

12. Verfahren gemäß Anspruch 10, wobei das genannte Drahtelement aus der Gruppe ausgewählt ist, bestehend aus einem Draht-Stent und einem Netz-Stent, der aus einem Metall-Schlauchmaterial ausgeschnitten ist und wobei der genannte Beschichtungsschritt des genannten Drahtelements weiterhin das Herstellen einer eingebauten Oberfläche umfasst, die die ganze Oberfläche des genannten Drahtelements umfasst.

13. Verfahren gemäß Anspruch 10, wobei der genannte Schritt des Zerstäubens von Tantalpentoxid mittels eines Vakuumplasma-Zerstäubens durchgeführt wird.

14. Verfahren gemäß Anspruch 10, wobei der genannte Beschichtungsschritt der genannten Drahtelemente weiterhin das Beschichten mit Polytetrafluorethylen umfasst.

## Revendications

1. Stent pouvant être inséré dans le corps d'un patient, ledit stent comprenant un fil métallique recouvert d'un revêtement ayant une structure en électret, ledit revêtement en électret comprenant une couche de pentoxyde de tantale recouvrant toute la surface dudit fil métallique.

2. Stent selon la revendication 1, qui est un stent vasculaire.

3. Stent selon la revendication 1, dans lequel ledit revêtement en pentoxyde de tantale est produit par un procédé de pulvérisation plasma sous vide.

4. Stent selon la revendication 1, dans lequel ledit revêtement comprend du polytétrafluoroéthylène.

5. Stent selon la revendication 2, ledit stent vasculaire étant constitué d'un tube découpé en treillis.

6. Stent selon la revendication 2, ledit stent vasculaire étant constitué d'au moins un fil métallique configuré en treillis.

7. Stent selon la revendication 6, dans lequel la distance entre deux fils métalliques adjacents individuels dudit stent est de moins de 15 diamètres de fil métallique, de telle sorte que ledit stent fournisse un champ électrostatique sensiblement uniforme empêchant l'agrégation des cellules sanguines sur lesdits éléments en treillis.

8. Stent selon l'une des revendications 6 ou 7, qui peut se déployer de telle sorte que les éléments en treillis soient exposés au revêtement en électret sans ombre.

9. Procédé de fabrication d'un stent portant une charge négative et pouvant être inséré dans le corps d'un patient, ledit procédé comprenant les étapes consistant à :
a. fournir un fil métallique ;
b. recouvrir ledit fil métallique avec un électret ;
ladite étape consistant à recouvrir ledit fil métallique avec un électret comprenant en outre le revêtement dudit fil métallique avec une couche de pentoxyde de tantale recouvrant toute la surface dudit fil métallique.

10. Procédé selon la revendication 9, dans lequel ledit fil métallique est monté sur un socle massif fournissant une dissipation efficace de la chaleur.

11. Procédé selon la revendication 10, dans lequel ledit stent est un stent vasculaire.

12. Procédé selon la revendication 10, dans lequel ledit fil métallique est choisi dans le groupe constitué d'un stent en fil métallique et d'un stent en treillis découpé dans un tube métallique et dans lequel ladite étape de revêtement dudit fil métallique comprend en outre la création d'une surface intégrale recouvrant toute la surface dudit fil métallique.

13. Procédé selon la revendication 10, dans lequel ladite étape consistant à pulvériser du pentoxyde de tantale est réalisée par pulvérisation plasma sous vide.

14. Procédé selon la revendication 10, dans lequel ladite étape de revêtement desdits fils métalliques comprend en outre le revêtement avec du polytétrafluoroéthylène.
